# EUROPEAN PATENT APPLICATION

(11) **EP 3 904 537 A2**
(43) Date of publication of application: **03.11.2021**
(21) Application number: 21171226.0
(22) Date of filing: 29.04.2021
(51) Int. Cl.: C12Q 1/70

(54) **METHOD OF IDENTIFYING SEVERE ACUTE RESPIRATORY SYNDROME CORONA VIRUS 2 (SARS-COV-2) RIBONUCLEIC ACID (RNA)**

(30) Priority: 29.04.2020 US 202063017043 P; 10.08.2020 US 202063063731 P; 30.12.2020 US 202017137972; 30.12.2020 US 202017138037
(71) Applicant: Teleflex Medical Incorporated, Morrisville, North Carolina 27560 (US)
(72) Inventor: BOUCHARD, Michael, Pennsylvania, 19610 (US); BRUZEK, Steven, Florida, 33703 (US); KNAPP, Chelsea, Florida, 33763 (US); MCDANIEL, Lauren, Florida, 33705 (US); SMALL, Jessica, Florida, 34677 (US); ULRICH, Robert, Florida, 34677 (US); WAGNER, Victoria, Michigan, 48306 (US)
(74) Representative: Jacob, Reuben Ellis

(57) **Abstract**

A method for detecting the presence of Severe Acute Respiratory Syndrome Coronavirus 2 (SARS-CoV-2) in a biological sample includes the steps of lysing the biological sample to form a lysate and generating an amplified lysate by performing a nucleic acid sequence-based (NASBA) amplification for a target nucleic acid sequence in the lysate in the presence of: a forward primer having the oligonucleotide sequence selected from the group consisting of SEQ ID NO: 1, SEQ ID NO: 5, SEQ ID NO: 9, SEQ ID NO: 13, and SEQ ID NO: 17; a reverse primer having the oligonucleotide sequence selected from the group consisting of SEQ ID NO: 2, SEQ ID NO: 6, SEQ ID NO: 10, SEQ ID NO: 14, and SEQ ID NO: 18; and a molecular beacon having the oligonucleotide sequence selected from the group consisting of SEQ ID NO: 3, SEQ ID NO: 7, SEQ ID NO: 11, SEQ ID NO: 15, and SEQ ID NO: 19 and a fluorophore. The amplified lysate is exposed to an excitation source. A fluorescence of the fluorophore is detected in the amplified lysate exposed to the excitation source. The SARS-CoV-2 is determined to be present in the biological sample in response to detecting the fluorescence of the fluorophore.

## Description

This invention relates generally to methods of identification of pathogenic microorganisms. More particularly, the present invention relates, for example, to a real-time method of identification of a pathogenic microorganism and/or its antibiotic resistance in a biological sample via nucleic acid sequence-based amplification (NASBA) of a specific RNA sequence.

Novel Coronavirus (SARS-CoV-2) responsible for Covid 19 is the cause of worldwide infections and is presently classified as a pandemic. While the full extent of Covid 19 morbidity and mortality may never be known, it is presently estimated to be in the millions worldwide. One important tool in decreasing the spread of the SARS-CoV-2 virus is fast and reliable testing due to the high prevalence of non-symptomatic carriers of the virus spreading the virus unknowingly. While there are some existing assays, these may take days to provide results. This long delay in results can allow additional infections to occur.

Thus, there remains a need for rapid and sensitive methods, preferably requiring minimal or no sample preparation, for detecting the presence of pathogen-associated analytes for identification of SARS-CoV-2 and subsequent diagnosis of Covid-19.

The foregoing needs are met, by the present invention, wherein aspects of a lysis solution and Covid-19 detection system and method are provided.

A first aspect of the disclosure pertains to a method for detecting the presence of Severe Acute Respiratory Syndrome Coronavirus 2 (SARS-CoV-2) in a biological sample. The method includes the steps of lysing the biological sample to form a lysate and generating an amplified lysate by performing a nucleic acid sequence-based (NASBA) amplification for a target nucleic acid sequence in the lysate in the presence of: a forward primer having the oligonucleotide sequence selected from the group consisting of SEQ ID NO: 1, SEQ ID NO: 5, SEQ ID NO: 9, SEQ ID NO: 13, and SEQ ID NO: 17; a reverse primer having the oligonucleotide sequence selected from the group consisting of SEQ ID NO: 2, SEQ ID NO: 6, SEQ ID NO: 10, SEQ ID NO: 14, and SEQ ID NO: 18; and a molecular beacon having the oligonucleotide sequence selected from the group consisting of SEQ ID NO: 3, SEQ ID NO: 7, SEQ ID NO: 11, SEQ ID NO: 15, and SEQ ID NO: 19 and a fluorophore. The amplified lysate is exposed to an excitation source. A fluorescence of the fluorophore is detected in the amplified lysate exposed to the excitation source. The SARS-CoV-2 is determined to be present in the biological sample in response to detecting the fluorescence of the fluorophore.

A second aspect of the disclosure pertains to a system for carrying out the above described method, the system comprising a lysing chamber, a NASBA fluidic network and an analytical instrument. The system may further comprise a sampling device.

According to a third aspect of the disclosure, the invention provides a reagent kit comprising a lysis solution,
a forward primer having the oligonucleotide sequence selected from the group consisting of SEQ ID NO: 1, SEQ ID NO: 5, SEQ ID NO: 9. SEQ ID NO: 13, and SEQ ID NO: 17;
a reverse primer having the oligonucleotide sequence selected from the group consisting of SEQ ID NO: 2, SEQ ID NO: 6, SEQ ID NO: 10, SEQ ID NO: 14, and SEQ ID NO: 18;
a molecular beacon having the oligonucleotide sequence selected from the group consisting of SEQ ID NO: 3, SEQ ID NO: 7, SEQ ID NO:11, SEQ ID NO: 15 and SEQ ID NO: 19;
and a fluorophore.
Particularly preferred combinations of primers and molecular beacons are;
SEQ ID NO: 1, SEQ ID NO: 2 and SEQ ID NO: 3
SEQ ID NO: 5, SEQ ID NO: 6 and SEQ ID NO: 7
SEQ ID NO: 9, SEQ ID NO: 10 and SEQ ID NO: 11
SEQ ID NO: 13, SEQ ID NO: 14 and SEQ ID NO: 15
SEQ ID NO: 17, SEQ ID NO: 18 and SEQ ID NO: 19

There has thus been outlined, rather broadly, certain embodiments of the invention in order that the detailed description thereof herein may be better understood, and in order that the present contribution to the art may be better appreciated. There are, of course, additional embodiments of the invention that will be described below and which will form the subject matter of the claims appended hereto.

In this respect, before explaining at least one embodiment of the invention in detail, it is to be understood that the invention is not limited in its application to the details of construction and to the arrangements of the components set forth in the following description or illustrated in the drawings. The invention is capable of embodiments in addition to those described and of being practiced and carried out in various ways. Also, it is to be understood that the phraseology and terminology employed herein, as well as the abstract, are for the purpose of description and should not be regarded as limiting.

As such, those skilled in the art will appreciate that the conception upon which this disclosure is based may readily be utilized as a basis for the designing of other structures, methods and systems for carrying out the several purposes of the present invention. It is important, therefore, that the claims be regarded as including such equivalent constructions insofar as they do not depart from the spirit and scope of the present invention.

In order that the invention may be readily understood, aspects of the invention are illustrated by way of examples in the accompanying drawings; however, the subject matter is not limited to the disclosed aspects.

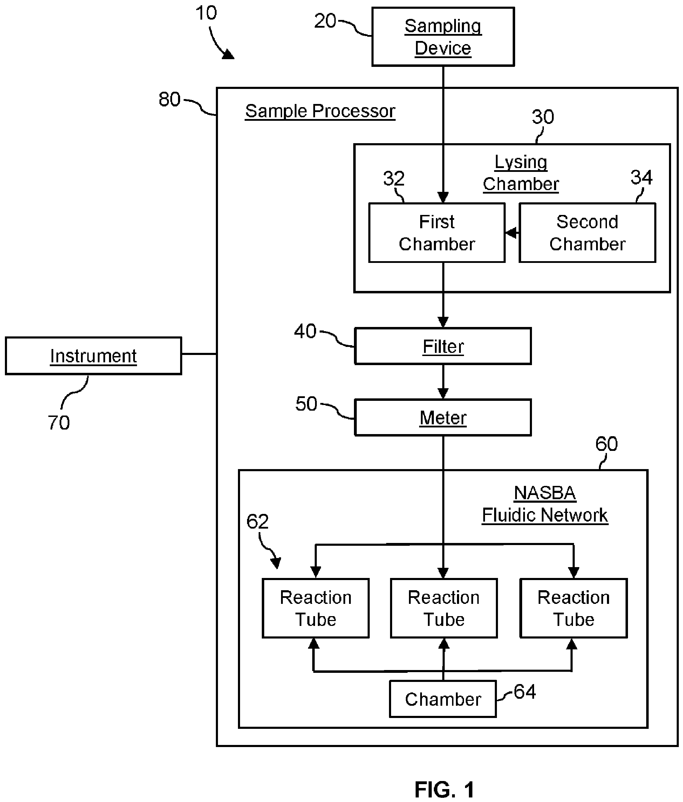

FIG. 1 illustrates a schematic infection detection system for use in and in accordance with aspects of the invention.

Features of the infection detection system according to aspects of the invention are described with reference to the drawings, in which like reference numerals refer to like parts throughout.

FIG. 1 shows a schematic representation of an exemplary SARS-CoV-2 infection detection system 10 in accordance with aspects of the invention. The SARS-CoV-2 infection detection system is configured to process a sample and to determine whether the sample contains one or more predetermined pathogens. The SARS-CoV-2 infection detection system in accordance with embodiments of the invention includes a sampling device 20, a lysing chamber 30, a filter 40, a meter 50, a nucleic acid sequence-based (NASBA) fluidic network 60, and an instrument 70. The SARS-CoV-2 infection detection system also includes a sample processor 80, such as a cartridge, which at least includes the NASBA fluidic network and may include any or all of the lysing chamber, the filter, and the meter. The sample processor is configured to connect to the sampling device and to receive and process a sample contained within the sampling device. The sample processor may be disposable and replaceable, and may be adapted to process the collected sample using at least one NASBA assay. The SARS-CoV-2 infection detection system may process the sample and determine whether the sample contains one or more predetermined pathogens rapidly, for example within an hour, thirty minutes, or less. The SARS-CoV-2 infection detection system may process the sample and determine whether the sample contains one or more predetermined pathogens at the point-of-care, for example within the same building, room, etc. as the patient. The SARS-CoV-2 infection detection system thus eliminates the need for time-wasting intermediary treatment, storage, and/or extraneous transport of the sampling device. According to aspects of the invention, the entirety of the sample processing may occur within the various components of the SARS-CoV-2 infection detection system thereby obviating the need of direct user intervention with the sample after the sample is collected. The SARS-CoV-2 infection detection system may accordingly be used by a user of low skill and may be readily transported to and applied in a variety of environments (e.g., the home, a hospital room, etc.). As a result, infection in a patient may be rapidly detected and identified, which may improve the prognosis of the patient.

In some embodiments, the sampling device of the SARS-CoV-2 infection detection system may be used in conjunction with the Centers for Disease Control (CDC) recommended commercial RNA extraction kits (or the like). The sampling device of the SARS-CoV-2 infection detection system may be adapted to collect a sample, such as nasopharyngeal swabs, saliva, sputum, blood (e.g., whole blood), urine, fecal matter, purulence/pus, etc. Whole blood, as used herein, means blood drawn directly from a patient from which none of the components, such as plasma, platelets, or pathogens, has been removed. The sampling device may collect the sample from a medical device (not shown). For example, the sampling device may be exposed for a predetermined and/or extended period of time to an internal space or lumen in the medical device so as to collect a sample of any pathogen which may form in said space and/or lumen. The medical device may be an external communicating device used for treating a patient, such as a Foley catheter, a vascular catheter, a suction catheter, a bronchial scope, a urinary drain line, a respiratory suction catheter, a Bronco-Alveolar-Lavage Catheter, etc. The sampling device may additionally or alternatively be adapted to collect a sample directly from a sample source such as nasopharyngeal swabs, saliva, sputum, urine, fecal matter, purulence/pus, a suspected infection site (such as a surgical dressing, wound, and/or an insertion site), etc. The sampling device may additionally or alternatively be adapted to collect a sample intravenously, subcutaneously, or intraosseously. The sampling device may be disposable and replaceable. According to aspects of the invention, the sampling device may include a sample collection tube. The sample collection tube may be a standard blood collection vacuum tube containing a whole blood sample. Additionally or alternatively, the sampling device may be a standard syringe containing a whole blood sample.

Depending on the source of the sample, lysing the sample is, optionally performed. For example, some samples such as nasopharyngeal swabs and/or saliva may not require lysing. If lysing is performed, the lysing chamber may be any chamber configured to receive the sample and lyse the sample into a lysate. The lysing chamber may be in fluid communication with the sampling device. Fluid communication, as used herein, may mean that the structures in question are fluidly connected via any of a number of structures such as tubing, conduits, etc., that allow fluid to travel from one structure to another. In embodiments of the invention, flow of the sample from the sampling device to the lysing chamber may be operatively connected to the instrument and may be controlled and/or driven by the instrument. Throughout this disclosure, reference is made to the instrument controlling and/or driving fluid flow, for example flow of the sample from the sampling device to the lysing chamber. The instrument may control and/or drive fluid flow when operatively connected to a fluid pathway and via any number of known fluid control systems, which may for example include pumps, valves, conduits etc. Further, the instrument may control and/or drive fluid flow without physically contacting the fluid. Accordingly, the sample collector and/or the sample processor maybe disposed and replaced while the instrument may be used repeatedly without contaminating the samples.

The lysing chamber may include all of the materials for lysing the sample and pathogen cells contained therein and for extracting and purifying pathogen messenger RNA (i.e., dissolve targeted mRNA and remove inhibitors that could interfere with nucleic acid amplification). For example, the lysing chamber may include a lysing agent, such as a lyophilized Acris lysing chemistry, that is configured to lyse the sample into the lysate. Additionally or alternatively, the lysing chamber may physically lyse the sample ultrasonically or by freezing the sample.

According to one aspect of the invention shown in FIG. 1, the lysing chamber may include a plurality of chambers, for example, a first chamber and a second chamber, The first chamber may include a lysing chemistry, such as, the lyophilized Acris lysing chemistry. The lysing chemistry contained within the first chamber may be in the form of a reagent plug(s) having dried lysis reagents. The first chamber may be in fluid communication with the sampling device and may receive the sample from the sampling device. In embodiments of the invention, the instrument may control and/or drive flow of the sample from the sampling device to the first chamber. Additionally or alternatively, the sample may be driven from the sampling device to the first chamber via gravity, capillary flow, etc. The second chamber may include a diluent and may be in fluid communication with the first chamber. The diluent may be driven from the second chamber to the first chamber to form the lysate. The instrument may control and/or drive the flow of the diluent from the second chamber to the first chamber. The lysate formed in the first chamber may contain the lysing agent, the diluent, and the sample. The diluent may be driven to the first chamber in advance of the arrival of the sample to prepare the lysate. Alternatively, the diluent and the sample may be driven to the first chamber simultaneously.

Depending on the sample source, filtering of the lysate is optionally performed. For example, nasopharyngeal swabs, saliva, etc., may not require filtering. Conversely, filtering whole blood may improve testing results. If filtering is performed, the filter is in fluid communication with the lysing chamber and is configured to filter the lysate into a filtered lysate. The filter may filter out large, opaque structures from the lysate (e.g., hemoglobin) while allowing a target sequence (e.g., genetic material from target pathogens) within the lysate to pass through the filter for subsequent processing and analysis. In embodiments of the invention, the instrument may control and/or drive the flow of the lysate from the lysing chamber and to through the filter to form the filtered lysate.

The meter is in fluid communication with the filter and is configured to meter a predetermined amount of filtered lysate for the NASBA analysis. The predetermined amount may, for example, be between 1 and 3 ml. In embodiments of the invention, the instrument may control and/or drive the flow of the filtered lysate from the filter and to the meter to collect the predetermined amount of filtered lysate.

The NASBA fluidic network may be in fluid communication with the meter and may receive the predetermined amount of filtered lysate from the meter. The NASBA fluidic network may include all of the materials (e.g., reagents, structures, etc.) necessary to perform predetermined NASBA-based nucleic-acid assays for mRNA and/or DNA on the predetermined amount of filtered lysate. The NASBA fluidic network may include a plurality of reaction tubes that are each directly or indirectly in fluidic communication with the meter and that are configured to receive filtered lysate from the meter. In embodiments of the invention, the instrument may control and/or drive flow of the filtered lysate from the meter to each of the plurality of reaction tubes.

Each of the plurality of reaction tubes may include all of the materials for processing the filtered lysate for isothermal amplification of predetermined pathogen target sequence (e.g., targeted mRNA to identify the presence of specific genes). Specific examples of materials that may be included in each of the plurality of reaction tubes include lysing buffers, mRNA-dependent DNA polymerase, mRNA primers, DNA primers, amino acids, and the like. Each of the plurality of reaction tubes may at least include an enzyme, a primer, and a beacon for performing an NASBA assay on a pathogen target sequence within the filtered lysate. Each of the plurality of reaction tubes may include one or more of the following three enzymes: Avian Myeloblastosis Virus (AMV) Reverse Transcriptase, a Ribonuclease H (RNase H), and a T7 RNA polymerase. Each of the plurality of reaction tubes may include two or more oligonucleotide primers. The enzyme(s) and the primer(s) may amplify a predetermined genetic sequence in the predetermined pathogen target sequence. The beacon provided in each of the plurality of reaction tubes may be configured to attach to the predetermined pathogen target sequence. The beacon may include a fluorophore that emits light when attached to the predetermined genetic sequence and when excited by an excitation source (e.g., a laser). Each reaction tube may include at least one window such that the instrument may detect light emitted from the beacon when attached to a predetermined pathogen target sequence. Each reaction tube may be provided with a beacon that is different from the beacons provided in each of the other reaction tubes. Accordingly, the NASBA fluidic network may detect as many different predetermined pathogen target sequences as there are reaction tubes.

The NASBA fluidic network may include a chamber containing an NASBA diluent. The chamber may be in fluid communication with each of the plurality of reaction tubes. In embodiments of the invention, the instrument may control and/or drive flow of the diluent from the chamber to each of the plurality of reaction tubes. The diluent contained within the chamber may be fluidly communicated to each of the plurality of reaction tubes a predetermined period (e.g., 5 minutes) before introduction of the filtered lysate. After expiration of the predetermined period, the filtered lysate may be distributed to each of the plurality of reaction tubes to induce the NASBA reactions and the results of the NASBA reactions may be analyzed by the instrument.

The instrument of the SARS-CoV-2 infection detection system may be adapted to receive the sample processor, to initiate and/or control aspects of processing of the sample within the sample processor, and to analyze the processed sample. As discussed in detail above, the instrument may control and/or drive fluid flow (e.g., whole blood flow, diluent flow, lysate flow, filtered lysate flow, etc.). In addition, the instrument may include a heater and/or a heat exchanger that may maintain the sample processor within a predetermined temperature range necessary for isothermal amplification of predetermined pathogen target sequences during the NASBA assays. The predetermined temperature range may be within 35-50 degrees Celsius. In embodiments, the predetermined temperature range may be within 40 - 42 degrees Celsius.

In embodiments of the invention, the instrument may be configured to perform any suitable NASBA-based nucleic-acid assay on the sample utilizing the reagents. For example, the instrument may be configured to perform any steps for lysing pathogen cells and extracting and purifying pathogen messenger RNA (i.e., dissolve targeted mRNA and remove inhibitors that could interfere with nucleic acid amplification). In another example, the instrument may be configured to perform any steps for processing the output solution from the extraction and purification steps for isothermal amplification of targeted mRNA to identify the presence of specific genes.

In accordance with a variety of embodiments of the present disclosure, pathogenic microorganisms and/or sequences related to antibiotic resistance are detected in a biological sample obtained from a patient. For the purposes of this disclosure, a biological sample includes nasopharyngeal swabs, saliva, sputum, whole blood, serum, plasma, cerebrospinal fluid (CSF), urine, synovial fluid, breast milk, sweat, tears, saliva, semen, feces, vaginal fluid or tissue, sputum, nasopharyngeal aspirate or swab, lacrimal fluid, mucous, or epithelial swab (buccal swab), and tissues (e.g., tissue homogenates), organs, bones, teeth, among others). For the purposes of this disclosure, a pathogenic microorganism includes, for example, one or more of *Betacoronavirus SARS-CoV-2 (Covid-19)* and/or other viruses in the family, *Coronaviridae.* More particularly, the pathogenic organisms may include those listed in Table I. More particularly still, the target sequences of the pathogenic organisms may be detected using the forward primer, reverse primer, and molecular beacon listed in Table I.

**Table I**

| Target | Gene name/target | Forward Primer | Reverse Primer | Molecular Beacon | Target Sequence |
|---|---|---|---|---|---|
| 2019 (SARS-CoV-2) | Nucleocapsi d protein N1 region | 5’- GACCCCAAAATCAGC GAAAT-3’ (SEQ ID NO: 1) | 5'- AATTCTAATACGACT CACTATAGGGAGAAC TCCATTCTGGTTACT GCCA-3' (SEQ ID NO: 2) | 5'-6- FAM/AGCTCGCATTAC GTTTGGTGGACCCTCG AGCT/3Dabcyl-3' (SEQ ID NO: 3) | 5'- GACCCCAAAATCAGCG AAATGCACCCCGCATT ACGTTTGGTGGACCCT CAGATTCAACTGGCAG TAACCAGA-3' (SEQ ID NO: 4) |
| 2019 (SARS-CoV-2) | Nucleocapsi d protein N2_{A} region | 5'- AAAACGTACTGCCAC TAAAGC-3' (SEQ ID NO: 5) | 5'- AATTCTAATACGACT CACTATAGGGAGAAT GTCTGATTAGTTCCT GGTC-3' (SEQ ID NO: 6) | 5'-6- FAM/AGCTCGCAGACG TGGTCCAGAACAAACG AGCT/3Dabcyl-3' (SEQ ID NO: 7) | 5'- TTTTGGGGACCAGGAA CTAATCAGACAAGGAA CTGATTACAAACATTG GCCGCAAATTGCACAA TTTGCCCCCAGCGCTT CAGCGTTCTTCGGAAT GTCG-3' (SEQ ID NO: 8) |
| 2019 (SARS-CoV-2) | Nucleocapsi d protein N2_{B} region | 5′- AATGTCTGGTAAAGG CCAAC-3′ (SEQ ID NO: 9) | 5'- AATTCTAATACGACT CACTATAGGGAGAAG CAGTACGTTTTTGCC GAGGC-3' (SEQ ID NO: 10) | 5'-6-FAM/ AGCTCGCCAAACTGTC ACTAAGAAATCGAGCT /3Dab-3' (SEQ ID NO: 11) | 5'- AATGTCTGGTAAAGGC CAACAACAACAAGGCC AAACTGTCACTAAGAA ATCTGCTGCTGAGGCT TCTAAGAAGCCTCGGC AAAAACGTACTGC-3' (SEQ ID NO: 12) |
| 2019 (SARS-CoV-2) | Nucleocapsi d protein N3 region | 5'- ACAAAGACGGCATCA TATGGG-3' (SEQ ID NO: 13) | 5'- AATTCTAATACGACT CACTATAGGGAGAAA TTGCAGCATTGTTAG CAGG-3' (SEQ ID NO: 14) | 5'-6FAM- AGCTCGGAATACACCA AAAGAYCACACGAGCT -Dabcyl-3' (SEQ ID NO: 15) | 5'- ACAAAGACGGCATCAT ATGGGTTGCAACTGAG GGAGCCTTGAATACAC CAAAAGATCACATTGG CACCCGCAATCCTGCT AACAATGCTGCAAT- 3' (SEQ ID NO: 16) |
| 2019 (SARS-CoV-2) | Small Envelope protein E | 5'- GTACGTTAATAGTTA ATAGC -3' (SEQ ID NO: 17) | 5'- AATTCTAATACGACT CACTATAGGGAGAAA TTGCAGCAGTACGCA CACA-3' (SEQ ID NO: 18) | 5'-6FAM- AGCTCGCTTTCGTGGT ATTCTTGCTAGTCGAG CT-Dab-3' (SEQ ID NO: 19) | 5'- GTACGTTAATAGTTAA TAGCGTACTTCTTTTT CTTGCTTTCGTGGTAT TCTTGCTAGTTACACT AGCCATCCTTACTGCG CTTCGATTGTGTGCGT ACTGCTGCAAT-3' (SEQ ID NO: 20) |
| Homo *sapiens* | Ribonucleas e P | 5'- GTTCTGACCTGAAGG CTCTG-3' (SEQ ID NO: 21) | 5'- AATTCTAATACGACT CACTATAGGGAGAAT CCTTAAAGTCAACGA TATG-3' (SEQ ID NO: 22) | 5’-6-FAM/ AGCTCGCTATTCAGTT GTTGCTATCACGAGCT /3Dabcyl-3’ (SEQ ID NO: 23) | 5'- GTTCTGACCTGAAGGC TCTGCGCGGACTTGTG GAGACAGCCGCTCACC TTGGCTATTCAGTTGT TGCTATCAATCATATC GTTGACTTTAAGGA - 3' (SEQ ID NO: 24) |

The forward primers, reverse primers, and molecular beacons listed in Table I are particularly suitable for use in the SARS-CoV-2 infection detection system 10. In this regard, these forward primers, reverse primers, and molecular beacons are optimized for use with a NASBA amplification and detection system.

A lysing solution suitable for use in the infection detection system 10 and method quickly lyses microbial cell wall and membranes. In a particular example, the lysing solution may facilitate this lysis at room temperature and without physio-mechanical cell disruption. In addition, the lysing solution may be benign to RNA and stable at room temperature. A specific example of a suitable lysing solution is found in Table II:

**Table II**

| Constituent | Concentration range |
|---|---|
| Quanidinium thiocyanate/guanidine thiocyanate | 2 mM to 16 mM |
| Tris HCL, pH 8.5 | 20 µM to 160 µM |
| Magnesium chloride | 6 µM to 48 µM |
| Potassium chloride | 35 µM to 280 µM |
| IGEPAL CA-630® | 0.1% v/v to 1.0% v/v |
| Nuclease Free Water | Bulk |

It is an advantage of the lysing solution according to Table II that it is suitable for use in lysing a variety of gram positive, gram negative, and fungal microorganisms. In addition, it is an advantage of the lysing solution according to Table II that it has a viable shelf life of greater than 1 year of storage at room temperature. In addition, it is an advantage of the lysing solution according to Table II that it has a viable shelf life of greater than 1 year of storage at negative twenty degrees Celsius (-20°C). Of note, IGEPAL CA-630® is a nonionic, non-denaturing detergent having the IUPAC name of octylphenoxypolyethoxyethanol.

The many features and advantages of the invention are apparent from the detailed specification, and thus, it is intended by the appended claims to cover all such features and advantages of the invention which fall within the true spirit and scope of the invention. Further, since numerous modifications and variations will readily occur to those skilled in the art, it is not desired to limit the invention to the exact construction and operation illustrated and described, and accordingly, all suitable modifications and equivalents may be resorted to, falling within the scope of the invention.

## Claims

1. A method for detecting the presence of Severe Acute Respiratory Syndrome Coronavirus 2 (SARS-CoV-2) in a biological sample, the method comprising:
lysing the biological sample to form a lysate;
generating an amplified lysate by performing a nucleic acid sequence-based (NASBA) amplification for a target nucleic acid sequence in the lysate in the presence of:
a forward primer having the oligonucleotide sequence selected from the group consisting of SEQ ID NO: 9, SEQ ID NO: 5, SEQ ID NO: 1, SEQ ID NO: 13, and SEQ ID NO: 17;
a reverse primer having the oligonucleotide sequence selected from the group consisting of SEQ ID NO: 10, SEQ ID NO: 6, SEQ ID NO: 2, SEQ ID NO: 14, and SEQ ID NO: 18; and
a molecular beacon having the oligonucleotide sequence selected from the group consisting of SEQ ID NO: 11, SEQ ID NO: 7, SEQ ID NO: 3, SEQ ID NO: 15, and SEQ ID NO: 19 and a fluorophore;
exposing the amplified lysate to an excitation source;
detecting a fluorescence of the fluorophore in the amplified lysate exposed to the excitation source; and
determining whether the SARS-CoV-2 is present in the biological sample in response to detecting the fluorescence of the fluorophore.

2. The method according to claim 1, wherein the forward primer has the oligonucleotide sequence consisting of SEQ ID NO: 9, the reverse primer has the oligonucleotide sequence consisting of SEQ ID NO: 10, and the molecular beacon has the oligonucleotide sequence consisting of SEQ ID NO: 11.

3. The method according to claim 1, wherein the forward primer has the oligonucleotide sequence consisting of SEQ ID NO: 5, the reverse primer has the oligonucleotide sequence consisting of SEQ ID NO: 6, and the molecular beacon has the oligonucleotide sequence consisting of SEQ ID NO: 7.

4. The method according to claim 1, wherein the forward primer has the oligonucleotide sequence consisting of SEQ ID NO: 1, the reverse primer has the oligonucleotide sequence consisting of SEQ ID NO: 2, and the molecular beacon has the oligonucleotide sequence consisting of SEQ ID NO: 3.

5. The method according to claim 1, wherein the forward primer has the oligonucleotide sequence consisting of SEQ ID NO: 13, the reverse primer has the oligonucleotide sequence consisting of SEQ ID NO: 14, and the molecular beacon has the oligonucleotide sequence consisting of SEQ ID NO: 15.

6. The method according to claim 1, wherein the forward primer has the oligonucleotide sequence consisting of SEQ ID NO: 17, the reverse primer has the oligonucleotide sequence consisting of SEQ ID NO: 18, and the molecular beacon has the oligonucleotide sequence consisting of SEQ ID NO: 19.

7. The method according to claim 1, further comprising:
adding a lysis solution to the biological sample to form the lysate, the lysis solution comprising:
2 mM to 16 mM of a Quanidinium thiocyanate/guanidine thiocyanate;
20 µM to 160 µM of a Tris HCL, pH 8.5;
6 µM to 48 µM of a Magnesium chloride;
35 µM to 280 µM of a Potassium chloride; and
0.1% v/v to 1.0% v/v of an octylphenoxypolyethoxyethanol.

8. The method according to claim 7, wherein the forward primer has the oligonucleotide sequence consisting of SEQ ID NO: 1, the reverse primer has the oligonucleotide sequence consisting of SEQ ID NO: 2, and the molecular beacon has the oligonucleotide sequence consisting of SEQ ID NO: 3.

9. The method according to claim 7, wherein the forward primer has the oligonucleotide sequence consisting of SEQ ID NO: 5, the reverse primer has the oligonucleotide sequence consisting of SEQ ID NO: 6, and the molecular beacon has the oligonucleotide sequence consisting of SEQ ID NO: 7.

10. The method according to claim 7, wherein the forward primer has the oligonucleotide sequence consisting of SEQ ID NO: 9, the reverse primer has the oligonucleotide sequence consisting of SEQ ID NO: 10, and the molecular beacon has the oligonucleotide sequence consisting of SEQ ID NO: 11.

11. The method according to claim 7, wherein the forward primer has the oligonucleotide sequence consisting of SEQ ID NO: 13, the reverse primer has the oligonucleotide sequence consisting of SEQ ID NO: 14, and the molecular beacon has the oligonucleotide sequence consisting of SEQ ID NO: 15.

12. The method according to claim 7, wherein the forward primer has the oligonucleotide sequence consisting of SEQ ID NO: 17, the reverse primer has the oligonucleotide sequence consisting of SEQ ID NO: 18, and the molecular beacon has the oligonucleotide sequence consisting of SEQ ID NO: 19.

13. A reagent kit for use in the method of any preceding claim, the kit comprising:
a lysis solution;
a forward primer having the oligonucleotide sequence selected from the group consisting of SEQ ID NO: 1, SEQ ID NO: 5, SEQ ID NO: 9. SEQ ID NO: 13, and SEQ ID NO: 17;
a reverse primer having the oligonucleotide sequence selected from the group consisting of SEQ ID NO: 2, SEQ ID NO: 6, SEQ ID NO: 10, SEQ ID NO: 14, and SEQ ID NO: 18;
a molecular beacon having the oligonucleotide sequence selected from the group consisting of SEQ ID NO: 3, SEQ ID NO: 7, SEQ ID NO:11, SEQ ID NO: 15 and SEQ ID NO: 19;
and a fluorophore.


<110>  Teleflex Medical Incorporated

<120>  METHOD OF IDENTIFYING SEVERE ACUTE RESPIRATORY SYNDROME CORONA
       VIRUS 2 (SARS-COV-2) RIBONUCLEIC ACID (RNA)

<130>  J111118EP

<150>  US 63/017,043
<151>  2020-04-29

<150>  US 63/063,731
<151>  2020-08-10

<150>  US 17/137,972
<151>  2020-12-30

<150>  US 17/138,037
<151>  2019-12-30

<160>  24

<170>  PatentIn version 3.5

<210>  1
<211>  20
<212>  DNA
<213>  Coronavirus SARS-CoV-2

<400>  1
gaccccaaaa tcagcgaaat                                                   20


<210>  2
<211>  49
<212>  DNA
<213>  Coronavirus SARS-CoV-2

<400>  2
aattctaata cgactcacta tagggagaac tccattctgg ttactgcca                   49


<210>  3
<211>  32
<212>  DNA
<213>  Coronavirus SARS-CoV-2

<400>  3
agctcgcatt acgtttggtg gaccctcgag ct                                     32


<210>  4
<211>  72
<212>  DNA
<213>  Coronavirus SARS-CoV-2

<400>  4
gaccccaaaa tcagcgaaat gcaccccgca ttacgtttgg tggaccctca gattcaactg       60

gcagtaacca ga                                                           72

<210>  5
<211>  21
<212>  DNA
<213>  Coronavirus SARS-CoV-2

<400>  5
aaaacgtact gccactaaag c                                                21


<210>  6
<211>  49
<212>  DNA
<213>  Coronavirus SARS-CoV-2

<400>  6
aattctaata cgactcacta tagggagaat gtctgattag ttcctggtc                  49


<210>  7
<211>  32
<212>  DNA
<213>  Coronavirus SARS-CoV-2

<400>  7
agctcgcaga cgtggtccag aacaaacgag ct                                    32


<210>  8
<211>  100
<212>  DNA
<213>  Coronavirus SARS-CoV-2

<400>  8
ttttggggac caggaactaa tcagacaagg aactgattac aaacattggc cgcaaattgc      60

acaatttgcc cccagcgctt cagcgttctt cggaatgtcg                           100


<210>  9
<211>  20
<212>  DNA
<213>  Coronavirus SARS-CoV-2

<400>  9
aatgtctggt aaaggccaac                                                  20


<210>  10
<211>  50
<212>  DNA
<213>  Coronavirus SARS-CoV-2

<400>  10
aattctaata cgactcacta tagggagaag cagtacgttt ttgccgaggc                 50


<210>  11
<211>  32
<212>  DNA
<213>  Coronavirus SARS-CoV-2

<400>  11
agctcgccaa actgtcacta agaaatcgag ct                                    32

<210>  12
<211>  93
<212>  DNA
<213>  Coronavirus SARS-CoV-2

<400>  12
aatgtctggt aaaggccaac aacaacaagg ccaaactgtc actaagaaat ctgctgctga      60

ggcttctaag aagcctcggc aaaaacgtac tgc                                   93


<210>  13
<211>  21
<212>  DNA
<213>  Coronavirus SARS-CoV-2

<400>  13
acaaagacgg catcatatgg g                                                21


<210>  14
<211>  49
<212>  DNA
<213>  Coronavirus SARS-CoV-2

<400>  14
aattctaata cgactcacta tagggagaaa ttgcagcatt gttagcagg                  49


<210>  15
<211>  32
<212>  DNA
<213>  Coronavirus SARS-CoV-2

<400>  15
agctcggaat acaccaaaag aycacacgag ct                                    32


<210>  16
<211>  94
<212>  DNA
<213>  Coronavirus SARS-CoV-2

<400>  16
acaaagacgg catcatatgg gttgcaactg agggagcctt gaatacacca aaagatcaca      60

ttggcacccg caatcctgct aacaatgctg caat                                  94


<210>  17
<211>  20
<212>  DNA
<213>  Coronavirus SARS-CoV-2

<400>  17
gtacgttaat agttaatagc                                                  20


<210>  18
<211>  49
<212>  DNA

<213>  Coronavirus SARS-CoV-2

<400>  18
aattctaata cgactcacta tagggagaaa ttgcagcagt acgcacaca                49


<210>  19
<211>  34
<212>  DNA
<213>  Coronavirus SARS-CoV-2

<400>  19
agctcgcttt cgtggtattc ttgctagtcg agct                                34


<210>  20
<211>  107
<212>  DNA
<213>  Coronavirus SARS-CoV-2

<400>  20
gtacgttaat agttaatagc gtacttcttt ttcttgcttt cgtggtattc ttgctagtta      60

cactagccat ccttactgcg cttcgattgt gtgcgtactg ctgcaat                  107


<210>  21
<211>  20
<212>  DNA
<213>  Homo sapiens

<400>  21
gttctgacct gaaggctctg                                                20


<210>  22
<211>  49
<212>  DNA
<213>  Homo sapiens

<400>  22
aattctaata cgactcacta tagggagaat ccttaaagtc aacgatatg                49


<210>  23
<211>  32
<212>  DNA
<213>  Homo sapiens

<400>  23
agctcgctat tcagttgttg ctatcacgag ct                                  32


<210>  24
<211>  94
<212>  DNA
<213>  Homo sapiens

<400>  24
gttctgacct gaaggctctg cgcggacttg tggagacagc cgctcacctt ggctattcag      60

ttgttgctat caatcatatc gttgacttta agga                                94